## Europäisches Patentamt
## European Patent Office
## Office européen des brevets

(19)

(11) Publication number: **0 131 789**
A2

(12)

# EUROPEAN PATENT APPLICATION

(21) Application number: **84107242.4**

(22) Date of filing: **23.06.84**

(51) Int. Cl.⁴: **C 07 K 15/00**
C 12 P 21/00, A 61 K 45/02
G 01 N 33/50, C 12 N 1/36
C 12 N 5/00
//(C12P21/00, C12R1/91),
(A61K45/02, 37/02)

(30) Priority: **27.06.83 US 508472**

(43) Date of publication of application:
**23.01.85 Bulletin 85/4**

(84) Designated Contracting States:
**AT BE CH DE FR GB IT LI LU NL SE**

(71) Applicant: **Sloan-Kettering Institute For Cancer Research**
**1275 York Avenue**
**New York New York 10021(US)**

(72) Inventor: **Old, Lloyd J.**
**600 West End Avenue**
**New York New York 10024(US)**

(72) Inventor: **Carswell Richards, Elizabeth**
**10 Otter Trail**
**Westport Connecticut 06880(US)**

(72) Inventor: **Rubin, Berish Y.**
**535 East 8th Street**
**New York New York 11218(US)**

(72) Inventor: **Williamson, Barbara D.**
**36 Shore Road**
**Old Greenwich Connecticut 06870(US)**

(72) Inventor: **Prendergast, Jay S.**
**9 Maple Avenue**
**Larchmont New York 10538(US)**

(74) Representative: **Patentanwälte Schulze Horn und Hoffmeister**
**Goldstrasse 36**
**D-4400 Münster(DE)**

(54) **Purified human tumour necrosis factor, method of producing it, composition comprising it in conjunction with human interferon and its use.**

(57) A method for the production and purification of human tumor necrosis factor (hTNF) is described. This purified material, shows growth inhibitory, hemorrhagic necrosis, cytotoxicity, and cytostasis effects on a variety of human tumors and cancers. The hTNF is free of exogeneous endotoxin, bacteria, and serum factors, hTNF has a molecular weight of about 70,000 hTNF and human interferon used together have a far greater growth inhibitory or cytotoxic effect on human tumors than the sum of their separate effects. The effect is synergistic and is seen with alpha-, beta- or gamma-IFN and with natural and recombinant IFN.

EP 0 131 789 A2

- 2 -

The present invention was wholly or partially made with funds provided by the National Cancer Institute under Grant No. CA 08748. Accordingly, the United States Government has certain rights in this invention.

This invention relates to a method of treating human cancer in humans using purified human tumor necrosis factor (hTNF) alone, or hTNF in conjunction with human interferon (hIFN). A method for producing hTNF is described.

### Background

#### TNF

The history of tumor necrosis factor (TNF) begins more than a century ago with the finding that during certain bacterial infections in humans, there was a concomittant regression of any human tumors present in vivo. For over 40 years, Coley and others treated human malignancies with bacterial cell-free filtrates or mixed bacterial vaccines often times with positive results.

- 3 -

Studies in guinea pigs and mice were done as well. The antitumor effect in experimental animals is a severe hemorrhagic reaction at the core of the tumor within 4 hrs of bacterial administration to a test subject or animal. This is followed by a slower necrotizing effect which exhibits a growing intensity over the next 48 hours. The tumor mass undergoes a progressive darkening in color indicative death and hemorrhage leading in many instances to complete tumor regression. This striking hemorrhagic necrosis induced in certain experimental tumors by gram negative bacteria or by endotoxin (lipopolysaccharide) derived from their cell wall has long been considered as the experimental counterpart of the clinical observations described above.

However, work at Sloan-Kettering led to the conclusion that bacterial stimulation causes release of a factor, possibly of macrophage origin, that is itself directly or indirectly responsible for tumor cell killing. Evidence for this comes from work showing that the serum of BCG-infected mice injected with endotoxin causes hemorrhagic necrosis of transplanted sarcoma Meth A and other tumors . Serum from BCG injected mice or serum from normal mice given endotoxin is inactive. Endotoxin alone lacks toxicity for most tumor cells in vitro. This active serum component has been called tumor necrosis factor (TNF).

- 4 -

TNF activity cannot be ascribed to residual endotoxin as measured by pyrogenicity, Limulus testing and chemical analysis for endotoxin moieties. TNF, unlike endotoxin, is directly cytotoxic for certain tumor cells in vitro; cultured cells from normal sources remain uninjured [Lloyd J. Old, New Developments in Cancer Therapy, MSKCC Clinical Bulletin 6:118 (1976), E.A. Carswell et al Proc. Nat'l. Acad. Sci. USA 72 3666-70 Sept 1975].Other rodents, i.e. rats and rabbits, produce TNF as well, with BCG plus endotoxin administration. TNF was first found in the serum of mice primed with Bacillus Calmette-Guérin (BCG), and challenged 2-3 weeks later with endotoxin from E. Coli. Neither BCG or its counterparts (see below), nor endotoxin alone produces the TNF factor in mouse serum; both must be present. C. granulosum, C Parvum, malaria or Zymosan (yeast cell walls), which like BCG, induce massive hyperplasia of macrophage and other cellular elements of the reticuloendothelial system can substitute for BCG in priming mice for release of TNF. These factors are BCG counterparts. The serum derived after BCG and endotoxin treatment is atypical in that it does not completely clot. A protocol for optimal production and assay of TNF in mice includes:

a) BCG: an inoculum of $2 \times 10^7$ viable BCG organisms for maximal stimulation and hyperplasia of the

- 4 -

-5-

reticulo-endothelial (RES) system,

b) endotoxin: 14-21 days later, at height of RES
stimulation by BCG, 0.25-25 microgram inoculum of
endotoxin (lipopolysaccharide w from E. Coli). The
highest dose gives the best TNF levels in mice,

c) TNF collection:

The optimal time is 2 hr post endotoxin
administration (later times less efficient due to
circulatory collapse produced by shock), and

d) TNF assays:

1) In Vivo:

The necrotic effect of TNF positive mouse serum on
BALb/c Meth A ascites Sarcoma transplanted into (BALb/c
x C57 BL/6) $F_1$ hybrid is demonstrated in the following
way. After 7 days the effect of TNF positive serum on
subcutaneous transplant tumor in vivo is scored
visually within 24 hr. A reaction is first seen in 3-4
hours. In 7-day transplants, the necrotic response
generally corresponds with the volume of TNF positive
serum administered from about 0.1-0.5 ml. +++ reaction
shows that the tumor mass has been completely or almost
completely destroyed leaving only a peripheral rim of
apparently viable tumor tissue. 6 day transplants are
less responsive, 5 day transplants not at all
responsive.

-6-

2)   In Vitro

TNF sensitive L-M cells were derived from
cloned mouse (L929) cells deposited at the American Type Cul-
ture Collection (ATCC) under the deposit number L 929. A TNF-
resistant line of L-cells was derived by repeated
passage of the TNF-sensitive L cells in media
containing TNF.   TNF is cytotoxic for TNF sensitive
L-cells [L(S)] but not for TNF resistant L-cells
[L(R)].

The assay is done using 0.5 ml of medium containing
serial dilutions of TNF added to wells (24 well Costar
plates) seeded 2-3 hours previously with 50,000
typsinized L-cells in 0.5 ml Eagle's minimal essential
medium and determining the the amount of protein that
resulted in 50% cell kill, estimated at 48 hours by
phase microscopy or trypan blue exclusion.   For
example, for one standard batch 1 unit in vitro
partially purified mouse TNF is equal to 58 ng or a
specificactivity of 20,000 units/mg protein.   This
partially purified mouse TNF contains no IFN.   The
original serum before purification has some Interferon
(IFN).

-Y-

Besides BCG,.C. granulosum, C. Parvum, Malaria or Zymosan (yeast cell wall) can also be used as priming agents. Green et al., J. Nat'l. Cancer Inst. $\underline{59}$:1519 (1977) report mice reach a maximum hypersensitivity to endotoxin six days post C. Parvum injection. In that method endotoxin administration begins 4 days after C. Parvum. Endotoxin doses are as little as 0.25 micro grams but 25 micrograms are used to elicite TNF release. TNF release is maximal 90 minutes after intravenous injection of 1 microgram of endotoxin. TNF production varies with different mouse stains.

Endotoxin from any gram negative bacterium such as E. Coli is the most efficient agent found to date to release TNF in vivo. (Carswell et al, Supra) The effect of TNF is seen in a variety of tumor (mouse) systems, namely: Sarcomas S-180 (CD-1 Swiss), BP8 (C3H); Leukemias EL4(C57Bl/6), ASL1 (A Strain), RADA-1 (A Strain), RLol (BALB/c), and mastocytoma P815 (DBA/2). Meth A is highly sensitive to TNF when the tumor is injected intradermally as in the in vivo assay. Tumors with lesser response were also reported: reticulum-cell sarcoma RCS5 (SJL) was resistant, Mammary tumors of (C3H/An x I) $F_1$ were only minimally responsive and AKR spontaneous leukemias were of intermediate sensitivity. Thus mouse TNF clearly has a therapeutic effect on mouse tumors. (Carswell et al, Supra).

In vitro, transformed murine fibroblasts (L cells) in culture were most sensitive to TNF as compared to Meth A sarcoma cells or mouse embryo fibroblasts (MEF). Viable cells are counted after 48 hour exposure to TNF. The effect here is cytotoxic as well as cytostatic, but delayed, since it is not seen for the first 16 hours. The effect in the Meth A in vivo test is a necrosis process which begins in the centre of the Meth A tumor spreading outward leaving a rim of seemingly unaffected tumor tissue. The tumor can then begin to grow again from this residuum. Treatment with an optimal dose of TNF will cause complete regression in a good percent of the animals so treated. The L cell effect is the basis for the in vitro assay, Supra. A resistant line of L-cells has been developed as well, derived by repeated passage of TNF-sensitive L-cells in media containing TNF. TNF is cytotoxic for sensitive L-cells [L(s)] but not for resistant L-cells (L(R)). These assays are also used for assay of hTNF (below).

Indirect indications so far point to macrophages as being one cell source of TNF in mice and rabbits since liver and spleen macrophages undergo massive hyperplasia when under the influence of BCG (or its counterparts) plus endotoxin.

283 JEL/HRL
6/2/83

- 9 -

In sum then: 1) agents which prime for TNF release cause
marked macrophage hyperplasia 2) selective lysis of splenic
macrophages is seen shortly after endotoxin injection into
BCG-primed mice 3) serum containing TNF is rich in enzymes
of lysosomal origin and activated macrophages are
characteristically rich in such lysosomes.

Cloned lines of mouse histiocytomas (J774 and PU5-1.8)
produce TNF constitutively; which production is greatly
increased after exposure to endotoxin.  [Mannel et al (1980)
Infect. Immun. 30, 523-530, and Williamson et al
unpublished].

As isolated from serum, mouse TNF is a glycoprotein
which exists in at least two forms, a 40-60,000 and a
150,000 molecular weight form. [Mannel et al (1980) Infect.
Immun. 28, 204-211; Kull et al. (1981) J. Immunol. 126,
1279-83; Haranaka, K. et al., (1981) Jpn J. Exp. Med., 51,
191-194; Green, S. et al. (1976) Proc. Nat'l. Acad. Sci. USA
73, 381-385 and unpublished]. TNF is stable when frozen,
preferably below - 70°C. Its activity is destroyed at 70°C
for 30 min. It is pyrogenic in rabbits in a range from 5-500 microgram/kg and non-pyrogenic at 5 microgram/kg.
Rabbit TNF has been reported to have a molecular weight range
of approximately 39-68 k daltons. [Ruff, M.R. et al. (1980)
J. Immunol. 125, 1671-1677; Matthews, N. et al. (1980) Br.
J. Cancer 42 416-422; Matthews, N. et al. (1978) Brit. J.
Cancer 38, 302-309; Ostrove, J.M. et al. (1979) Proc. Soc.
Exp. Biol. Med. 160, 354-358]

## Interferon

Proteinaceous interferon is produced by host animals as
a result of a previous virus infection. The host interferon
produced protects the animal against a subsequent viral
infection. IFN has been shown to play an important role in
the immunoregulatory system. Interferon has been found to
effect the following cell functions: It inhibits cell
division, tumor growth, antibody formation, erythroid
differentiation, and adipocytes differentiation while
enhancing macrophage phagocytosis, lymphocyte toxicity, NK
cell activity, cell surface antigen expression and antibody
production. Interferon (IFN) has recently been found to be
a possible anti-cancer agent as well.

- II -

There are three main interferon types namely alpha,
beta and gamma, distinguished by their antigenic and
biochemical properties.  Alpha is secreted by leukocyte
white blood cells, beta by fibroblasts and both of these can
be virally induced.Gamma IFN is secreted by lymphoid cells
and is known as "immune" interferon.  [Stewart, W.E. II, et
al., Nature 286, 110 (1980)]  Major antigenic differences
form the primary basis for differentiation among the three
IFN species.  In addition, the IFNs differ in a number of
biological and physicochemical properties.  Human alpha and
beta IFN have been purified and their amino acid sequences
have been determined partly by direct amino acid sequencing
[Knight, E. Jr., et al., Science 207, 525-526 (1980); Zoon,
K.C., Science 207, 527-528 (1980)] and more completely by
analysis of the cloned complementary DNA sequences [Mantei,
N., et al.  Gene 10, 1-10 (1980); Taniguchi, T., et al.,
Gene 10, 11-15 (1980)].  Comparison of cloned alpha- and
beta-IFN DNA sequences indicated only 29% homology between
the two IFNs at the amino acid level [Taniguichi, T.,
Mantei, N., et al., Nature 285, 547-549 (1980)].  Within

-12.

each of the three major IFN species there may be molecular subspecies with a lesser degree of heterogeneity.  Several subspecies of human alpha-IFN have been recognized so far by comparison of cloned DNA sequences.  [Nagata, S., et al.,

Nature 287 401-408 (1980)].


Much less information is available about gamma-IFN.

This IFN is usually found in supernatants of lymphocyte cultures exposed to mitogens -- such as various lectins or specific antigens in cultures of sensitized cells.  The definition of gamma-IFN rests on two major criteria: (i) unlike alpha- and beta-IFN, the biological activity of gamma-IFN is largely destroyed by exposure to pH 2, and (ii) antisera prepared against alpha and and beta IFN do not cross-react with gamma-IFN.  In addition, experiments carried out with relatively crude preparations of gamma-IFN suggest a number of biological differences.  For example, gamma-IFN was shown to induce the antiviral state much more slowly than alpha or beta-IFN [Dianzani, F., et al., Nature 283, 400-402 (1980)].  In contrast, gamma-IFN might be more active as a cell growth inhibitory and anti-tumor agent

- 13

[Crane, J.L. Jr., et al., J. Nat. Cancer Insti. 61 871-874 (1973) Gupta et al, Proc. Nat'l. Acad. Sci. U.S.A. 76, 4817 (1979) Blalock et al, Cellular Immunology 49:390-394 (1980)].


Clinical trials of IFN for cancer therapy had been hindered because not enough could be produced. Genetic engineering techniques have surmounted this problem and the National Cancer Institute is conducting large scale trials of human interferon (hIFN). Thus both natural and recombinant hIFNs are known and used clinically.


## Summary


The present invention reveals a process for the production and purification of human TNF (hTNF) for the first time. Such a process would be useful for clinical trials. Over 30 human cell lines in tissue culture are screened for their ability to produce TNF. Phorbol 12-myristate, 13-acetate (PMA) is also found to be necessary to enhance hTNF production. PMA is known as a tumor promoting agent. B cell lines with or without PMA are the

- 14 -

most promising sources for hTNF production, at the present time. The standard TNF assays described above are used to assay for hTNF i.e. the *in vivo* assay for tumor necrosis and the *in vitro* assay for L-cell cytostasis/cytotoxicity. T cells, monocyte, and promylocyte cell lines produce little or no TNF. Neither exogenous BCG, nor exogenous endotoxin, are necessary for hTNF production by this method. A surprising synergistic anti-tumor cell effect is found when hTNF and either recombinant or natural hIFN are used together to kill or inhibit human tumor cells. hIFN or hTNF exhibit an anti-tumor effect individually, but the effect of hTNF used together with hIFN is greater than the sum of their separate anti-tumor effects.

## Description

The present invention finds that human cell lines can serve as the source of hTNF. Methods of production of mouse TNF seem to be mostly dependent on the effects of BCG and endotoxin. The present method of production from human B-cells produces TNF without added BCG or endotoxin. Thus, this TNF is essentially free of exogenous endotoxin, bacterial, and as well as serum contaminants. As shown in Table I, of the hematopoietic cells tested, B cells are the best producers of TNF.  High levels of TNF production
without the presence of either exogenous BCG or exogenous
endotoxin is an unexpected result.  Small or trace amounts
of hTNF are found in supernatants of cell lines of T-cell,
monocyte or pro-myelocytic origin.  The in vitro L-cell
assay (Carswell et al, Supra) determined levels of TNF
produced by cells of human origin.  Thus for the first time,
levels of TNF found in this study are free of any exogenous
endotoxin contamination since there is no endotoxin-addition
to the culture system as described below. Previous studies
by others in the mouse find the presence of endotoxin a
problem as it can be difficult to free a system of
endotoxin.

EBV transformed B cell lines are derived from patients
with melanoma.  Other cell lines are from cell bank
collections of Dr. Lloyd Old, Dr. Jorgen E. Fogh or Dr.
Peter Ralph of Sloan-Kettering Institute.  LukII cells are
obtained from Dr. W. Stewart (Pickering et al (1980) Proc.
Nat'l. Acad. Sci. USA 77, 5938-5942). EBV-transformed B cell
lines are derived from patients with melanoma (Houghton
et al, Proc. Nat'l. Acad. Sci. USA, 77 4260 (1980).

**0131789**

- 16

In screening human cells of hematopoietic origin for TNF production, (see Table I) $5 \times 10^5$ cells per ml are cultured in RPMI 1640 containing 8% fetal calf serum (FCS) with and without PMA [10 ng/ml (Sigma Chemical Co., St. Louis, Mo)]. Table 2 shows the composition of RPMI 1640. After incubation for 48 hrs at 37°C, the cells are collected by centrifugation and resuspended at $1 \times 10^6$ per ml in RPMI 1640 medium without PMA for another 48 hrs. Cell-free supernatants are collected by centrifugation of the cell cultures. These supernatants are frozen at -20°C before TNF activity is determined by the L cell assay used on supernatants diluted by one-half.

In the _in vitro_ assay, mouse L-M cells derived from the NCTC clone 929 (L) line were obtained from the American Type Culture Collection (ATCC) and grown in Eagle's Minimum Essential Medium (MEM) (GIBCO, Grand Island, N.Y.) supplemented with nonessential amino acids, penicillin (100 U/ml), streptomycin (100 g/ml) and 8% heat-inactivated fetal calf serum, at 37°C. A mouse TNF-resistant L cell line L(R) was derived from sensitive L cells L(S) by repeated passages in mouse TNF-containing medium. The activity of hTNF is assayed by adding equal volumes of medium (such as 0.5 ml)

· 17·

containing serial dilutions of TNF to wells (24-well Costar
plates) seeded three hours previously with 50,000
trypsinized L cells (in 0.5 ml of medium), and determining
the amount of protein that results in 50% cell kill
estimated at 48 hours by phase microscopy, or trypan blue
exclusion.

As seen in Table I, hTNF as produced by human B-cells
is cytotoxic for mouse L(S) cells but not mouse L(R) cells
in the standard mouse TNF in vitro assay as described above.
Cells to be screened for hTNF are grown with and without
tumor growth-promoting agent.  PMA, for example, is
effective in enhancing several fold the hTNF response of
B-cells as shown in the L(S) columns of Table I [see column
heading "no PMA"].  PMA is also known as TPA.

+PMA

Cultured mouse L-M cells derived from L(929) (ATCC)
made resistant to hTNF by repeated passage in medium
containing hTNF (2-3 microgram every week continuously to
about $10^6$ cells) are also  completely cross-resistant to
mouse TNF.  This is an interesting cross-resistance effect.

- 18 -

To assay for hTNF In Vivo, [BALB/c x C57BL/$_6$]F$_1$ ♀ mice (Jackson Labs), injected intradermally 7 days earlier with 5 x 10$^5$ Meth A sarcoma cells, receive a single intravenous dose of hTNF. After 24 hr, tumor hemorrhagic necrosis is graded as: no effect (-), slight (+), moderate (++), or extensive (+++), the latter involving 90% of the tumor surface (see Carswell Supra). Thus hTNF shows a similar effect on standard TNF assays in vivo and in vitro, though in general hTNF has a higher specific activity on human cell targets as compared to mouse TNF.

Endotoxin is determined by Limulus Amebocyte Lysate (Microbiological Associates).

Since cloned lines of mouse histiocytes produce TNF, further studies of normal and malignant human histiocytes obviously must be done to determine production of hTNF by these cells in humans. The examples of B-cells from Table I suggest normal B cells have this capacity as well.

Example of Preparation and Concentration of hTNF (from LukII Cells). 5 x 10$^5$ LukII cells/ml are cultured in RPMI 1640 containing 5% FCS and 10 ng/ml PMA and incubated

- 18 -

- 19 -

for 48 hr. at 37°C.  At 48 hr, cells are collected by centrifugation and resuspended at 8-10 x $10^5$/ml in serum-free R-ITS PREMIX [Insulin (5 micrograms/ml), Transferrin (5 micrograms/ml), selenum (5ng/ml) serum substitute (Collaborative Research, Waltham Mass)] and 2 nM ethanolamine and incubated an additional 48 hr. Supernatants are spun free of cells and frozen at -20°C. LukII supernatants are concentrated by Amicon stirred cells (PM 10 membrane), and applied to a DEAE-Sephadex A-50 column (40 x 2.6 cm) equilibrated with Tris 0.05M, NaCl 0.15 M buffer, pH 7.3 at 5°C.  5 ml fractions are eluted with the same buffer.  TNF activity is detected in the initial flow-through fractions and such peak fractions are pooled. This procedure results in a 25-fold increase in specific activity (2 - 5 x $10^4$ units/mg protein).  hTNF-active fractions as determined by the L-cell in vitro assay are pooled and concentrated by Amicon cells.

The specific activity of the pooled fractions of hTNF is in the approximate range of $1 \times 10^3$-$1 \times 10^4$ in the case of cells grown in media supplemented with fetal calf serum (FCS).  The specific activity of the pooled fractions of hTNF is in the approximate range of $2$-$5 \times 10^4$ micrograms in the case of cells grown in serum-free media.

- 20 -

I.V. injection of DEAE fractionated hTNF caused hemorrhagic necrosis of Meth A tumors by the in vivo test, Supra. 300 micrograms produced +++ necrosis in 1/5 of the mice and ++ necrosis in the remaining 4/5. 150 microgram of hTNF produced ++ necrosis in 5/7 of the mice and + necrosis in 2/7 of the mice. 75 micrograms of hTNF produced ++ necrosis in 1/5 of the mice and + necrosis in 4/5 mice. No necrosis is observed in three out of three (3/3) control mice following injection of comparable DEAE fractions of culture medium (RPMI 1640). Thus hTNF is fully active in vivo and in vitro in standard tests used to determine TNF activity.

Whether grown with or without FCS, the molecular weight of hTNF is approximately 70,000 daltons as determined on Sephacryl S-200 column chromatography. High or low pH over for example, 12 hrs., destroys the activity of hTNF; e.g. approximately 90% loss of activity at pH 2.0, 55% activity is destroyed at pH 4.0, and approximately 45% loss at pH 10. Activity of hTNF is stable in the pH range of approximately 6-8. As to heat stability, hTNF activity is destroyed by exposure to 70°C for 60 min. but is stable at 56°C for 60 min. Activity is preserved even after long periods of storage at -78°C or -20°C.

- 21 -

IFN activity of 100 units per ml is found in the unfractionated supernatant from PMA-stimulated LUKII cells. No interferon activity is detected in the pooled fractions of purified hTNF derived by DEAE chromatography, nor in the purified mouse TNF used. Human interferon activity is measured by inhibition of the cytopathic effect of vesicular stomatis virus on WISH or GM 2767 cells [Stewart W.E. II (1979) The Interferon System (Springer, Vienna] and compared against an international standard in the case of recombinant human alpha-IFN (Hoffman- La Roche) and the laboratory standard in the case of human natural gamma-IFN. Recombinant alpha-hIFN ($2-4 \times 10^8$ units/mg protein) is obtained from Hoffman-La Roche, natural alpha-hIFN (leukocyte-derived) ($0.64 \times 10^6$ units/mg protein) is prepared for the Sloan-Kettering Institute evaluation program by the Kocher Laboratory, Berne Switzerland, natural beta-hIFN ($1 \times 10^7$ units/mg protein) is obtained from Roswell Park Memorial Institute (RPMI) and natural gamma-hIFN (leukocyte-derived) ($1 \times 10^7$ units/mg protein) is obtained from Dr. Berish Rubin, Sloan-Kettering Institute. Neither mouse nor human TNF as partially purified have detectable IFN activity. The various hIFNs lack detectable hTNF activity as shown by lack of cytotoxicity or cytostasis on L(S) cells in the in vitro assay, Supra.

The hTNF produced has different effects on different cell lines as shown in Table 3. Cytotoxicity is noted using a 35% or greater reduction in cell viability after seven days while cytostasis is noted using a 35% or greater reduction in cell number after seven days. For this study and for all further studies as shown in this description hTNF as produced by the LUKII cell line as described above is used. This specific set of examples is for reference purposes only, and is not meant to limit the invention. Other B-cell line hTNF, as well as TNF from other cell sources in humans, can obviously be used on a variety of human cells _in vivo_ and _in vitro_. It will be obvious to those skilled in the art to see the effect of BCG and its counterparts as well as endotoxin on the TNF production of human cells _in vitro_ and _in vivo_.

The effect of hTNF on human breast cell lines (Tables 3 & 4) is similar to that of mouse TNF since both it are cytotoxic for a majority of the cell lines of breast cancer origin tested. Lung and melanoma cells are also affected by hTNF but here the predominant effect is cytostatic. Of the four normal cell lines tested with hTNF, lung, kidney, fetal lung and fetal skin, none are sensitive to hTNF. Therefore, the cytotoxic, cytostatic or hemorrhagic necrosis effect of

0131789

- 23

hTNF on human cells appears to be confined to human tumor cells.  hTNF has a higher specific activity on human cell targets as compared to mouse TNF.

Human interferon, whether recombinant or natural, whether alpha, beta or gamma, has an inhibitory effect on tumor cell growth as is well known and, as shown here, by example for four tumor cell lines Tables 5, 6 and 9.  In general, the number of anti-viral units of IFN required to cause a 35% or greater cytotoxic or cytostatic effect is higher with alpha-hIFN than with beta or gamma-hIFN.  None of the IFN preparations showed cytotoxicity or cytostasis on L(S) mouse cells in vitro, thereby indicating absence of hTNF activity.  Yet these two cellular products when purified and used together, exhibit an effect greater than the sum of their separate effects as seen in the examples shown in tables 7 and 8.  The combined cytotoxic effect of IFN and hTNF is found also to be synergistic by the method suggested by Clarke (Clark, D.A. (1958) Ann. N.Y. Acad. Sci. 76 915-921) i.e. pharmacologically synergistic.  See Tables 10, 11 and 12.  This unexpected result can be of enormous value in treating human tumors.

-24-

An example of a method used to exhibit the effect of hTNF, hIFN or a combination of the two follows. Cells are trypsinized, rinsed twice, plated at 4-5 x $10^4$/well (24 well Costar plates) in MEM containing 8% FCS and incubated at 37°C. After 16-24 hr, the medium is aspirated and 1 ml dilution of either DEAE-fractionated hTNF, or IFN, or hTNF plus IFN is then added. Total cell counts (viable and non-viable) are determined at 3, 5 and 7 days by phase microscopy. Cultures are refed on day 5 with fresh medium containing the same concentrations of hTNF and/or hIFN. This method is used for the results in Tables 3-9. The protocol according to Clark, Supra to screen for drug synergism or potentiation is to test two factors, X and Y herein hIFN(X) and hTNF(Y) alone and in combination. Enhanced tumor inhibition or cell toxicity leads to trials of ½X +½Y or herein, ½ hIFN +½ hTNF to see if this quarter-dose combination is comparable to the full dose of either alone and if it is, synergism of the two factors X and Y, herein hIFN and hTNF is demonstrated. This we see in Tables 10, 11 and 12 wherein the same method as for table examples 4-8 is used at days 3 and 5 only. Clearly, synergism is shown at ½ hIFN + ½ hTNF which effect is greater than either TNF alone or hIFN alone for the three cell lines in tables 10, 11 and 12.

**0131789**

Thus the two, hTNF and hIFN, can be used above or together in various combinations to treat human tumors and cancer since they elicit a greater cytotoxic activity on cancer together than either one alone. Indeed, it is obvious that to obtain maximal therapeutic effect, cancer patients should be stimulated to produce both TNF and/or IFN, or treated with varying combinations of the above therapeutic species (TNF and IFN), perhaps alternating them for maximal benefit, or using different timed sequences etc. If other factors such as TNF are necessary for maximal IFN action, supply of this deficiency can lead to enhanced IFN activity against cancer. IFN augmented action may be due to hTNF functioning as a biological response modifier of the immune system. It is then obvious to those skilled in the art to try hIFN action against human cancer cells in conjunction with other biological response modifiers alone or in combination, such as hormones, or other immune response modifier molecules which are hormone-like, such as IL-2, or other cytokines, or lymphokines, or lymphotoxin. Indeed testing varying combinations of the different IFNs and such molecules against cancer is indicated. To these methods can be added testing of hTNF or hIFN from different cell sources against cancer.

## TABLE I

### SCREENING OF HUMAN CELL LINES OF HEMATOPOIETIC ORIGIN FOR hTNF PRODUCTION
### L Cell % Viability In Vitro

| Cell Line | L (S) | | No PMA | L (R) | |
|---|---|---|---|---|---|
| | No PMA | + PMA | + PMA | No PMA | + PMA |
| **B cell** | | | | | |
| BE* | 47% | 20% | 2.3 | 100% | 100% |
| BI* | 57% | 43% | 1.3 | 100% | 98% |
| CL* | 95% | 37% | 2.6 | 100% | 97% |
| CW* | 28% | 8% | 3.5 | 100% | 100% |
| DE* | 45% | 18% | 2.5 | 100% | 100% |
| DM* | 18% | 11% | 1.6 | 100% | 100% |
| DS* | 88% | 38% | 2.3 | 100% | 100% |
| EJ* | 27% | 16% | 1.7 | 100% | 100% |
| EL* | 70% | 16% | 4.4 | 100% | 100% |
| EQ* | 15% | 11% | 1.4 | 100% | 100% |
| ER* | 47% | 22% | 2.1 | 100% | 100% |
| FC* | 19% | 8% | 2.4 | 100% | 100% |
| FD* | 44% | 16% | 2.8 | 97% | 100% |
| FG* | 80% | 30% | 2.7 | 100% | 97% |
| ARH-77 | 68% | 12% | 5.7 | 100% | 100% |
| DAUDI | 97% | 97% | — | N.T. | N.T. |
| LuK II | 20% | 13% | 1.5 | 100% | 100% |
| RPMI 1788 | 78% | 35% | 2.2 | 100% | 100% |
| RPMI 8226 | 96% | 84% | 1.1 | 100% | 100% |
| RPMI 8866 | 32% | 10% | 3.2 | 100% | 86% |
| SK-LY-16 | 98% | 100% | — | 100% | 98% |
| SK-LY-18 | 99% | 100% | — | 100% | 98% |
| ARA-10 | 77% | 39% | 2.0 | 100% | 97% |
| BALL-1 | 100% | 100% | — | 100% | 100% |
| **T Cell** | | | | | |
| CCRF-CEM | 100% | 100% | — | 100% | 100% |
| P-12 | 95% | 94% | — | 99% | 98% |
| MOLT-4 | 100% | 100% | — | 99% | 96% |
| T-45 | 100% | 100% | — | 100% | 100% |
| HPB-ALL | 100% | 97% | — | 100% | 100% |
| TALL-1 | 96% | 94% | — | 96% | 96% |
| **Monocyte** | | | | | |
| J111 | 100% | 100% | — | 99% | 98% |
| THP | 96% | 80% | — | 99% | 99% |
| U-937 | 100% | 100% | — | 99% | 99% |
| **Promyelocyte** | | | | | |
| HL-60 | 96% | 97% | — | 99% | 98% |

*EBV-transformed cell lines derived from peripheral blood.

TABLE 2

RPMI 1640
Liquid (IX)

0131789

| Component | mg/L |
|---|---|
| **INORGANIC SALTS:** | |
| $Ca(NO_3)_2 \cdot 4H_2O$ | 100.00 |
| KCl | 400.00 |
| $MgSO_4 \cdot 7H_2O$ | 100.00 |
| NaCl | 6000.00 |
| $NaHCO_3$ | 2000.00 |
| $Na_2HPO_4 \cdot 7H_2O$ | 1512.00 |
| **OTHER COMPONENTS:** | |
| D-Glucose | 2000.00 |
| Glutathione (reduced) | 1.00 |
| **AMINO ACIDS:** | |
| L-Arginine (free base) | 200.00 |
| L-Asparagine | 50.00 |
| L-Aspartic acid | 20.00 |
| L-Cystine | 50.00 |
| L-Glutamic acid | 20.00 |
| L-Glutamine | 300.00 |
| Glycine | 10.00 |
| L-Histidine (free base) | 15.00 |
| L-Hydroxyproline | 20.00 |
| L-Isoleucine (Allo free) | 50.00 |
| L-Leucine (Methionine free) | 50.00 |
| L-Lysine HCl | 40.00 |
| L-Methionine | 15.00 |
| L-Phenylalanine | 15.00 |
| L-Proline (Hydroxy L-Proline free) | 20.00 |
| L-Serine | 30.00 |
| L-Threonine (Allo free) | 20.00 |
| L-Tryptophan | 5.00 |
| L-Tyrosine | 20.00 |
| L-Valine | 20.00 |
| **VITAMINS:** | |
| Biotin | 0.20 |
| D-Ca pantothenate | 0.25 |
| Choline chloride | 3.00 |
| Folic acid | 1.00 |
| i-Inositol | 35.00 |
| Nicotinamide | 1.00 |
| Para-aminobenzoic acid | 1.00 |
| Pyridoxine HCl | 1.00 |
| Riboflavin | 0.20 |
| Thiamine HCl | 1.00 |
| Vitamin $B_{12}$ | 0.005 |

### TABLE 3

### EFFECT OF hTNF ON HUMAN CELL LINES

#### Cytotoxic

SK-MG-4 (Astrocytoma)
MCF-7 (Breast Ca)
BT-20 (Breast Ca)
SK-BR-3 (Breast Ca)
ME-180 (Cervix Ca)
SK-CO-1 (Colon Ca)
RPMI 7931 (Melanoma)

#### Cytostatic

SK-LU-1 (Lung Ca)
RPMI 4445 (Melanoma)
SK-MEL-29 (Melanoma)
SK-MEL-109 (Melanoma)
SK-OV-3 (Ovary Ca)

#### No Effect

T-24 (Bladder Ca)
5637 (Bladder Ca)
MDA-MB-361 (Breast Ca)
S-48 (Colon Ca)
SK-LC-4 (Lung Ca)
SK-LC-6 (Lung Ca)
SK-LC-12 (Lung Ca)
SK-MEL-19 (Melanoma)
SK-UT-1 (Uterus Ca)
SAOS-2 (Osteogenic Sarcoma)
U2OS (Osteogenic Sarcoma)
WI-38 (Normal Fetal Lung)
MY (Normal Kidney Epithelium)
F-136-35-56 (Normal Fetal Lung)
F-136-35-56 (Normal Fetal Skin)

-29-

## TABLE 4

### THE EFFECT OF hTNF ON FOUR HUMAN TUMOR CELL LINES
(DAY 0 - 5 x $10^4$ CELLS PLATED WITH hTNF)

| Cell Line | hTNF micrograms protein/culture | | DAY 3 | | DAY 5 | | DAY 7 | |
|---|---|---|---|---|---|---|---|---|
| | | | % viable cells | cell Growth x$10^5$ | %viable cells | cell Growth x$10^5$ | %viable cells | cell Growth x$10^5$ |
| | | TNF units | | | | | | |
| | 48 | 1263 | 24% | 1.03 | 16% | 1.55 | 5% | 1.45 |
| | 19.2 | 505 | 30 | 1.08 | 19 | 1.43 | 7 | 1.38 |
| BT-20 | 9.6 | 253 | 45 | 1.05 | 21 | 1.53 | 10 | 1.3 |
| (Breast) | 4.8 | 126 | 49 | 1.03 | 30 | 1.6 | 18 | 1.23 |
| (cyto- | 1.2 | 32 | 50 | 1.05 | 53 | 1.8 | 61 | 2.43 |
| toxic) | Control | 0 | 93 | .99 | 94 | 2.23 | 96 | 3.39 |
| | 24.5 | 645 | 83 | 1.78 | 52 | 2.7 | 49 | 6.33 |
| | 9.8 | 258 | 83 | 1.73 | 57 | 2.83 | 49 | 7.38 |
| ME-180 | 4.9 | 129 | 80 | 1.75 | 70 | 3.48 | 58 | 8.03 |
| (cervix) | 2.45 | 63 | 81 | 1.73 | 76 | 4 | 63 | 8.23 |
| (cyto-toxic) | Control | 0 | 98 | 1.62 | 97 | 4.48 | 97 | 1.01 |
| | 24.5 | 645 | 91 | .8 | 86 | 1.63 | 72 | 1.68 |
| SK-MEL- | 9.8 | 258 | 96 | 1.15 | 90 | 1.93 | 91 | 2.68 |
| 109 | 4.9 | 129 | 94 | 1.35 | 93 | 2.13 | 90 | 2.93 |
| (Melanoma) | 2.45 | 63 | 94 | 1.25 | 93 | 2.1 | 92 | 3.13 |
| (cytosta-tic) | Control | 0 | 98 | 2.19 | 98 | 3.89 | 99 | 9.78 |
| | 48 | 1263 | 95 | 2.38 | 98 | 4.6 | 99 | 7.75 |
| T-24 | 19.2 | 505 | 96 | 2.83 | 97 | 4.55 | 98 | 8.58 |
| (Bladder | 9.6 | 253 | 97 | 2.78 | 98 | 4.65 | 98 | 8.33 |
| no effect) | 4.8 | 126 | 98 | 3.25 | 99 | 4.55 | 98 | 8.33 |
| | Control | 0 | 100 | 3.23 | 98 | 4.53 | 99 | 8.38 |

## TABLE 5

### THE EFFECT OF RECOMBINANT HUMAN ALPHA INTERFERON ON FOUR HUMAN CELL LINES
(DAY 0 − 5x10$^4$ CELLS PLATED WITH IFN)

| Cell Line | IFN units per culture | DAY 3 | | DAY 5 | | DAY 7 | |
|---|---|---|---|---|---|---|---|
| | | % viable cells | cell Growth # cells x10$^5$ | %viable cells | cell Growth # cells x10$^5$ | %viable cells | cell Growth # cells x10$^5$ |
| BT-20 | 50,000 | 83 | .6 | 31 | .65 | 28 | .9 |
| | 12,500 | 85 | .65 | 47 | .95 | 38 | .925 |
| | 3,125 | 83 | .72 | 74 | 1.15 | 65 | 1.37 |
| | 781 | 83 | .75 | 75 | 1.32 | 70 | 1.4 |
| | Control | 95 | 1.04 | 93 | 2.3 | 97 | 5.4 |
| ME-180 | 50,000 | 85 | 1.02 | 78 | 1.67 | 62 | 2 |
| | 12,500 | 91 | 1.18 | 86 | 1.92 | 79 | 3.4 |
| | 3,125 | 90 | 1.3 | 91 | 2.02 | 87 | 4.2 |
| | 781 | 95 | 1.42 | 93 | 2.7 | 90 | 4.9 |
| | Control | 98 | 1.87 | 98 | 5.91 | 98 | 7.8 |
| SK-MEL-109 | 50,000 | 62 | .6 | 15 | 1.35 | 10 | 1.25 |
| | 12,500 | 58 | .65 | 34 | 1.77 | 37 | 2.1 |
| | 3,125 | 67 | 1.12 | 52 | 1.8 | 54 | 2.4 |
| | 781 | 79 | 1.42 | 69 | 2.37 | 74 | 2.5 |
| | Control | 97 | 2.2 | 98 | 3.6 | 98 | 6.8 |
| T-24 | 50,000 | 95 | 1.6 | 94 | 4.1 | 83 | 4.8 |
| | 12,500 | 94 | 1.7 | 96 | 4.7 | 89 | 5.3 |
| | 3,125 | 94 | 1.98 | 95 | 4.8 | 90 | 5.9 |
| | 781 | 97 | 2.72 | 94 | 4.97 | 90 | 7.4 |
| | Control | 98 | 3.43 | 96 | 6.05 | 93 | 7.97 |

TABLE 6

THE EFFECT OF NATURAL HUMAN GAMMA INTERFERON
ON FOUR HUMAN CELL LINES
(DAY 0 - 5x10$^4$ CELLS PLATED WITH IFN)

| Cell Line | -hIFN units per culture | DAY 3 % viable cells | DAY 3 cell Growth # cells x10$^5$ | DAY 5 %viable cells | DAY 5 cell Growth # cells x10$^5$ | DAY 7 %viable cells | DAY 7 cell Growth # cells x10$^5$ |
|---|---|---|---|---|---|---|---|
| BT-20 | 250 units | 73 | 1.33 | 79 | 2.2 | 58 | 2.83 |
| | 125 units | 81 | 1.43 | 81 | 2.43 | 66 | 3.15 |
| | 31.25 units | 84 | 1.78 | 86 | 2.7 | 79 | 4.03 |
| | 7.8 units | 86 | 1.93 | 93 | 2.78 | 87 | 3.83 |
| | Control | 95 | 1.64 | 97 | 3.61 | 97 | 5.44 |
| ME-180 | 250 units | 67 | 1.15 | 49 | 1.23 | 22 | 1.95 |
| | 125 units | 69 | 1.2 | 56 | 1.3 | 26 | 1.9 |
| | 31.25 units | 72 | 1.32 | 61 | 1.3 | 34 | 1.78 |
| | 7.8 units | 79 | 1.55 | 67 | 1.38 | 50 | 2.65 |
| | Control | 98 | 2.45 | 98 | 5 | 96 | 7.75 |
| SK-MEL-109 | 250 units | 55 | 1 | 51 | 1.73 | 23 | 2.3 |
| | 125 units | 67 | 1 | 61 | 2 | 38 | 2.78 |
| | 31.25 units | 74 | 1.18 | 82 | 3.02 | 61 | 3.35 |
| | 7.8 units | 85 | 1.15 | 90 | 3.28 | 77 | 5.45 |
| | Control | 98 | 2.05 | 98 | 3.53 | 96 | 5.43 |
| T-24 | 2000 units | 100 | 1.75 | 95 | 2.58 | 93 | 2.48 |
| | 500 units | 100 | 2.08 | 95 | 2.52 | 94 | 3.1 |
| | 125 units | 99 | 2.4 | 95 | 2.9 | 96 | 3.52 |
| | 31.25 units | 99 | 3.03 | 98 | 4.95 | 98 | 6.45 |
| | 7.8 units | 99 | 3.35 | 98 | 7 | 98 | 9.45 |
| | Control | 99 | 4 | 98 | 7.35 | 99 | 10.4 |

## TABLE 7

THE EFFECT OF hTNF AND RECOMBINANT HUMAN ALPHA INTERFERON
ON FOUR HUMAN TUMOR CELL LINES
(DAY 0 - 5 x $10^4$ CELLS PLUS hTNF AND IFN)

|  |  | % VIABLE CELLS | | |
|---|---|---|---|---|
|  |  | DAY 3 | DAY 5 | DAY 7 |
| BT-20 | hTNF + IFN* | 26 | 6 | 5 |
|  | hTNF 4 U | 74 | 64 | 62 |
|  | IFN 3125 U | 83 | 74 | 65 |
|  | Control | 95 | 93 | 97 |
| ME-180 | hTNF + IFN* | 53 | 25 | 20 |
|  | hTNF 16 U | 85 | 82 | 70 |
|  | IFN 3125 U | 90 | 91 | 87 |
|  | Control | 98 | 98 | 98 |
| SK-MEL-109 | hTNF + IFN* | 33 | 19 | 25 |
|  | hTNF 33 U | 96 | 96 | 96 |
|  | IFN 781 U | 79 | 69 | 74 |
|  | Control | 97 | 98 | 98 |
| T-24 | hTNF + IFN* | 97 | 93 | 85 |
|  | hTNF 33 U | 98 | 95 | 88 |
|  | IFN 781 U | 94 | 95 | 90 |
|  | Control | 98 | 96 | 93 |

(*Same amounts of hTNF and hIFN used in combination as used alone for each
cell lines).
U = Units.

## TABLE 8

THE EFFECT OF hTNF AND NATURAL HUMAN GAMMA INTERFERON
ON FOUR HUMAN TUMOR CELL LINES
(DAY 0 - 5 x 10$^4$ CELLS & hTNF AND hIFN)

|  |  | DAY 3<br>% viable cells | DAY 5<br>% viable cells | DAY 7<br>% viable cells |
|---|---|---|---|---|
| BT-20 | hTNF + IFN* | 11 | 10 | 6 |
|  | hTNF 25 U | 56 | 46 | 31 |
|  | IFN 31.25 U | 84 | 86 | 79 |
|  | Control | 95 | 97 | 97 |
| ME-180 | hTNF + IFN* | 2 | 4 | 5 |
|  | hTNF 50 U | 77 | 72 | 60 |
|  | IFN 125 U | 69 | 56 | 26 |
|  | Control | 98 | 98 | 96 |
| SK-MEL-109 | hTNF + IFN* | 9 | 6 | 2 |
|  | hTNF 100 U | 95 | 93 | 92 |
|  | IFN 125 U | 67 | 61 | 38 |
|  | Control | 98 | 98 | 96 |
| T-24 | hTNF + IFN* | 99 | 94 | 94 |
|  | hTNF 200 U | 97 | 97 | 96 |
|  | IFN 125 U | 99 | 97 | 93 |
|  | Control | 99 | 98 | 93 |

(*Same amounts of hTNF and hIFN used in combination as used alone for each cell lines).

U = units

0131789

Table 9 ·   The Cytotoxic and Cytostatic Effect of Human Interferons on Five Human Cell Lines.

Number of Anti-Viral Units Causing 35% Cytotoxicity or Cytostasis.

Cell Lines

| Human Interferon | BT-20 | | ME-180 | | SK-MEL-109 | | T-24 | | WI-38 | |
|---|---|---|---|---|---|---|---|---|---|---|
| | CT | CS | CT | CS | CT | CS | CT | CS | CT | CS |
| Natural IFN-α (alpha) | 270 | -25 | 6500 | 110 | 1250 | 26 | >50,000 | 1,900 | >50,000 | 230 |
| Recombinant IFN-α (alpha) | 3125 | <781 | 44,000 | <781 | 1400 | <781 | >50,000 | 16,000 | >50,000 | >50,000 |
| Natural IFN-β (Beta) | <49 | <49 | 260 | <49 | <49 | <49 | >12,500 | <49 | >25,000 | 160 |
| Natural IFN-γ (Gamma) | 130 | 72 | <7.8 | <7.8 | 22 | 27 | >2000 | 25 | >2000 | >2000 |

CT - Cytotoxic

CS - Cytostatic

# TABLE 10

## BT-20 SYNERGISM EXPERIMENT WITH NATURAL GAMMA-hIFN PLUS hTNF

|  | IFN (Units/culture) | DAY 3 # Cells x$10^5$ | %viable | DAY 5 # Cells x$10^5$ | % viable |
|---|---|---|---|---|---|
| *X* | 250 | 1.08 | *74* | 1.4 | *64* |
| ½ X | 125 | 1.03 | 76 | 1.83 | 79 |
| ¼ X | 62.5 | .95 | 84 | 1.73 | 83 |
|  | **hTNF (Units/culture)** |  |  |  |  |
| *Y* | 50 | 1.15 | *48* | 1.75 | *37* |
| ½ Y | 25 | 1.1 | 52 | 1.98 | 47 |
| ¼ Y | 12.5 | .98 | 59 | 2.28 | 54 |
|  | **IFN + hTNF (50 units)** |  |  |  |  |
| X + ½ Y | 250 | .75 | 3 | 1.05 | 5 |
| ½ X + ½ Y | 125 | .75 | 10 | 1.05 | 5 |
| ¼ X + Y | 62.5 | .7 | 7 | 1.05 | 9 |
|  | **IFN + hTNF (25 units)** |  |  |  |  |
| X + ½ Y | 250 | .85 | 12 | 1.05 | 5 |
| ½ X + ½ Y | 125 | .73 | 14 | 1 | 7 |
| ¼ X + ½ Y | 62.5 | .75 | 13 | .8 | 12 |
|  | **IFN + hTNF (12.5 units)** |  |  |  |  |
| X + ¼ Y | 250 | .8 | 22 | 1.13 | 11 |
| ½ X + ½ Y | 125 | .75 | 17 | 1.15 | 11 |
| *Synergism* *¼ X + ¼ Y* | 62.5 | .67 | *22* | 1.15 | *11* |
|  | Control | 1.02 | 96 | 1.97 | 94 |

## TABLE 11

### SK-MEL-109 SYNERGISM EXPERIMENT WITH NATURAL GAMMA-hIFN PLUS hTNF

### Natural Gamma-hIFN

| | IFN (Units/culture) | DAY 3 # Cells $\times 10^5$ | %viable | DAY 5 # Cells $\times 10^5$ | % viable |
|---|---|---|---|---|---|
| X | *250* | 1.2 | *58* | 1.08 | *32* |
| | 125 | 1.25 | 70 | 1.15 | 43 |
| | 62.5 | 1.2 | 72 | 1.5 | 63 |
| | **hTNF (Units/culture)** | | | | |
| Y | *200* | .8 | *87* | 1.38 | *87* |
| | 100 | .8 | 91 | 1.63 | 92 |
| | 50 | 1.15 | 91 | 1.73 | 96 |
| | **IFN + hTNF (200 units)** | | | | |
| | 250 | .875 | 8 | .9 | 3 |
| | 125 | .8 | 12 | .85 | 6 |
| | 62.5 | .775 | 16 | .83 | 6 |
| | **IFN + hTNF (100 units)** | | | | |
| | 250 | .825 | 12 | .83 | 3 |
| | 125 | .8 | 16 | .925 | 8 |
| | 62.5 | .825 | 18 | .95 | 16 |
| | **IFN + hTNF (50 units)** | | | | |
| | 250 | .8 | 19 | .85 | 6 |
| | 125 | 1 | 32 | .9 | 11 |
| *Synergism* ¼ X + ¼ Y | *62.5* | .875 | *31* | .9 | *14* |
| | Control | 1.44 | 96 | 4.12 | 99 |

283 JEL/HRL
5/18/83
0131789

- 37 -

## TABLE 12

### ME-180 SYNERGISM EXPERIMENT WITH NATURAL GAMMA-hIFN PLUS hTNF

#### Natural Gamma-hIFN

| | IFN (Units/culture) | DAY 3 # Cells $\times 10^5$ | %viable | DAY 5 # Cells $\times 10^5$ | % viable |
|---|---|---|---|---|---|
| X | *250* | 1.15 | *67* | 1.15 | *56* |
| | 125 | 1.3 | 71 | 1.4 | 64 |
| | 62.5 | 1.33 | 75 | 1.35 | 67 |
| | **hTNF (Units/culture)** | | | | |
| Y | *200* | 2.48 | *81* | 4.2 | *55* |
| | 100 | 2.38 | 86 | 4.4 | 59 |
| | 50 | 2.4 | 89 | 4.4 | 63 |
| | **IFN + hTNF (200 units)** | | | | |
| | 250 | .775 | 6 | 1.25 | 4 |
| | 125 | .775 | 6 | 1.15 | 2 |
| | 62.5 | .775 | 6 | 1.25 | 4 |
| | **IFN + hTNF (100 units)** | | | | |
| | 250 | .83 | 3 | 1.2 | 2 |
| | 125 | .8 | 3 | 1.23 | 2 |
| | 62.5 | .8 | 12 | 1.15 | 2 |
| | **IFN + hTNF (50 units)** | | | | |
| | 250 | .875 | 6 | 1.23 | 0 |
| | 125 | .85 | 12 | 1.05 | 2 |
| *Synergism* *½ X + ½ Y* | 62.5 | .8 | *16* | 1.08 | *7* |
| | Control | 2.69 | 98 | 5.61 | 98 |

- 37 -

Claims:

1. Purified hTNF.

2. hTNF of Claim 1 having a molecular weight in the range of about 70,000 daltons.

3. hTNF of Claim 1 stable in the approximate pH range of 6 - 8.

4. hTNF of Claim 1 having a specific activity in the range of 2 - 5 x $10^4$ units/mg Protein as determined by the mouse L-cell in vitro assay.

5. hTNF of Claim 1 stable at $-78^\circ$ C and $-20^\circ$ C.

6. hTNF of Claim 1 stable at $56^\circ$ C for 60 min.

7. Method for producing purified hTNF which comprises:
   (i)    incubating human hematopoietic cells initially in tissue culture;
   (ii)   separating the cell culture supernatant from said cells,
   (iii)  collecting said cells,
   (iv)   resuspending said cells and
   (v)    re-incubating them;
   (vi)   collecting the cell supernatant; and
   (vii)  freezing the supernatant without said cells.

8. Method of Claim 7 wherein the initial incubation of step (i) is in RPMI 1640 medium containing FCS in a range of about 0 - 8 %.

9. Method of Claim 7 wherein the cells are reincubated in step (iv) in RPMI 1640 medium.

10. Method of Claim 7 wherein the hematopoietic cells are human B cells.

11. Method of Claim 10 wherein the human B cells are selected from the group of BE, BI, CL, CW, DE, DM, DS, EJ, EL, EQ, ER, FC, FD, FG, ARH-77, DAUDI, LUKII, RPMI 1788, RPMI 8226, RPMI 8866 and ARA-10.

12. Method of Claim 7 wherein the cells are grown in step (i) in the presence of PMA.

13. Method of Claim 12, wherein after the initial incubation of step (i) said cells are resuspended in serum free R-ITS PREMIX and 2nM ethanolamine, the cell-free supernatants are collected and concentrated, said concentrated cell supernatant is contacted with a separation column equilibrated with buffer at pH 7.3 and the hTNF-active fractions obtained from said column are pooled and concentrated.

14. Composition comprising hTNF and hIFN.

15. Composition of Claim 14 wherein the hTNF is produced by the method of Claim 7.

16. Composition of Claim 14 wherein the hTNF is produced by the method of Claim 12.

17. Composition of Claim 14 wherein the hTNF is produced by the method of Claim 13.

18. Composition of Claim 14 wherein the hIFN is selected from the group of alpha-hIFN, beta-hIFN, gamma hIFN and mixtures thereof.

19. Composition of Claim 14 wherein the hIFN is natural hIFN.

20. Composition of Claim 14 wherein the hIFN is recombinant hIFN.

21. Method for distinguishing normal from malignant cells in a mixture which comprises contacting said cells with hTNF and observing a growth inhibitory effect on the malignant cells.

22. Method of Claim 21 wherein hTNF is produced according to the process of Claims 7 - 13.

23. Method of disthinguishing normal from malignant cells in a mixture which comprises contacting said cells with a composition comprising hTNF and hIFN and observing a growth inhibitory effect on the malignant cells.

24. Method of Claim 23 wherein hTNF is produced according to the process of Claims 7 - 13.

25. Method of Claim 23 wherein the hIFN is selected from the group of alpha-hIFN, beta-hIFN, gamma-hIFN and mixtures thereof.

26. Method of Claim 25 wherein the hIFN is natural hIFN.

27. Method of Claim 25 wherein the hIFN is recombinant hIFN.

28. Method for producing mouse L(R) cells resistant to hTNF by repeated passage of mouse L(S) cells through medium containing hTNF.

29. Method of Claim 28 wherein the hTNF is produced by the method of Claims 7 - 13.

30. Method for inhibiting growth of human cancer cells which comprises exposing the human cancer cells to amounts of hTNF sufficient to inhibit growth of said cells.

31. Method of Claim 30 wherein the hTNF is produced by the method of Claims 7 - 13.

32. Method for inhibiting growth of human cancer cells which comprises exposing the human cancer cells to a composition comprising of hTNF and hIFN sufficient to inhibit growth of said cells.

33. Method of Claim 32 wherein hTNF is produced according to the process of Claims 7 - 13.

34. Method of Claim 32 wherein the hIFN is selected from the group of alpha-hIFN, beta-hIFN, gamma-hIFN and mixtures thereof.

35. Method of Claim 34 wherein the hIFN is natural hIFN.

36. Method of Claim 34 wherein the hIFN is recombinant hIFN.

37. Purified hTNF of claim 1 as produced by the method of claims 7-13.